(19) Europäisches Patentamt
European Patent Office
Office européen des brevets

(11) Publication number: 0 066 346 B1

(12) EUROPEAN PATENT SPECIFICATION

(45) Date of publication of patent specification: 30.10.85

(51) Int. Cl.$^4$: C 07 D 207/323

(21) Application number: 82200660.7

(22) Date of filing: 29.05.82

# (54) Process for the preparation of a 2-alkylpyrrole.

(30) Priority: 02.06.81 NL 8102656

(43) Date of publication of application:
08.12.82 Bulletin 82/49

(45) Publication of the grant of the patent:
30.10.85 Bulletin 85/44

(84) Designated Contracting States:
BE CH DE FR GB IT LI NL

(56) References cited:
DE-C- 699 032

JOURNAL OF ORGANIC CHEMISTRY, vol. 23, September 1958, pages 1281-1286, Mack Printing Co., Easton, Pennsylvania, USA

JOURNAL OF THE CHEMICAL SOCIETY, 1948, pages 186-188, London, G.B.

CHEMISCHE BERICHTE, vol. 95, 1962, pages 307-318, Verlag Chemie GmbH, Weinheim, DE.

(73) Proprietor: STAMICARBON B.V.
Postbus 10
NL-6160 MC Geleen (NL)

(72) Inventor: Van der Stoel, Roland Emile
Schout Boutenstraat 27
NL-6121 HC Buchten (NL)
Inventor: Janssen, Petrus Hubert Joseph
Mauritspark 32
NL-6163 HN Geleen (NL)
Inventor: Van de Moesdijk, Cornelis Gerardus Maria
Drossaert Saldenstraat 7
NL-6181 ER Elsloo (NL)

(74) Representative: Hoogstraten, Willem Cornelis Roeland et al
OCTROOIBUREAU DSM Postbus 9
NL-6160 MA Geleen (NL)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for the preparation of a 2-alkylpyrrole. Such compounds are important, among other things, for the paint industry and for the preparation of agricultural chemicals.

It is known in the art that 2-methylpyrrole with a yield of 10—20% is formed in the catalytic reduction of levulinic acid nitrile in the liquid phase with hydrogen under high pressure (see the DE—C—699.032). In addition to the 2-methylpyrrole, 2-methylpyrrolidine with a yield higher than that of the 2-methylpyrrole is formed in this reduction process. For the preparation of 2-methylpyrrole this known process is hardly suitable, because the yield is rather low and a very high hydrogen pressure must be applied.

From the Journal of Organic Chemistry, *23* (1958) pp. 1281—1286, Mack Printing Co., Easton, Pa., USA a reductive cyclization of levulinic acid nitrile under low pressure in the presence of Raney nickel is known. Pyrroline and/or pyrrolidine are thus formed. A similar process is known from the Journal of Organic Chemistry, (1948) pp. 186—188, London, where aryl-substituted levulinic acid nitriles undergo reductive cyclization. Further it is known from Chemische Berichte *95* (1962) pp. 307—318 that α-cyano-levulinic acid ethyl esters may be cyclized reductively to yield a mixture of cyclization products. Neither of these references relate to gas phase processes.

It has now been found that 2-methylpyrrole and other 2-alkylpyrroles can be very well prepared with a higher yield and while applying a substantially lower hydrogen pressure than in the case of the said known process.

The process according to the invention for the preparation of a 2-alkylpyrrole of formula (I)

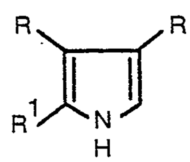

(I)

wherein $R^1$ represents an alkyl group with 1—3 C-atoms and the two Rs represent, independently of each other, H or an alkyl group with 1—2 carbon atoms by reaction of a 4-oxoalkanenitrile of formula (II)

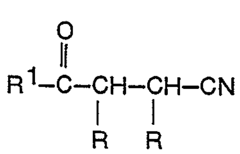

(II)

wherein $R^1$ and R have the meanings mentioned above in the presence of $H_2$ and a catalyst containing a metal selected from Cu, Ni, Pt, Pd or Rh or a compound thereof, optionally containing a promoter and optionally deposited on a supporting material at elevated temperatures, is characterised in that the reaction takes place in the gas phase at a temperature of 150—350°C.

If in applying the process according to the invention levulinic acid nitrile is started from, 2-methylpyrrole is obtained. If, for instance, 2,3-dimethyl-4-oxopentanenitrile is taken as starting compound, 2,3,4-tri-methylpyrrole will be formed.

Preference is given to the use of a catalyst containing a metal or compound of a metal from the group of platinum, palladium and rhodium.

The catalysts can be applied on a supporting material, such as activated carbon, graphite, silicon oxide, zinc oxide, aluminium oxide, magnesium oxide and mixtures of these materials. As supporting material aluminium oxide is particularly suitable. A promotor can be added to the catalyst as well. As such an alkali metal is preferably used. The amount of catalyst on the carrier may usefully vary from 0.1—30 wt.% (calculated as metal and based on the total amount of catalyst material including the carrier).

The process according to the invention is carried out at a temperature of 150—350°C. preferably a temperature of between 175 and 300°C is applied.

The process according to the invention can be carried out as known in the art for the performance of catalytic gas phase reactions, for instance by passing the gaseous starting product, diluted if so desired with an inert gas such as nitrogen, together with hydrogen, over the catalyst in the form of a fixed bed while applying a space velocity of between, for instance, 0.03 and 2 g starting product per ml catalyst (bulk volume) per hour.

The process according to the invention can be carried out with different quantities of hydrogen, for instance a quantity of 1—50 moles hydrogen per mole starting product. More than 50 moles hydrogen per mole starting product can be applied as well, but this does not result in an advantage.

The 4-oxoalkanenitrile to be applied in the process according to the invention can be prepared as known in the art, for instance by reaction of hydrogen cyanide with an α,β-unsaturated ketone (see Methoden der Organischen Chemie HOUBEN-WEYL *8* pp. 272—274).

By cooling the gaseous reaction mixture obtained in applying the process to the invention separation can be obtained into a condensate and hydrogen-containing gas, which can be recirculated. Besides the 2-alkylpyrrole, this condensate may contain a small quantity of the corresponding 5-alkylpyrrolidone-2, for instance 5-methylpyrrolidone-2 if levulinic acid nitrile is started from. The condensate obtained can be separated by, for instance, fractional distillation.

In the following examples the invention will be further elucidated.

### Example I

Through a vertical tubular reactor (18 mm diameter, 400 mm length) containing a zone of 25 ml (bulk volume) catalyst a vaporous mixture of levulinic acid nitrile and hydrogen (6 moles hydrogen per mole nitrile) is passed from top to bottom at atmospheric pressure for 3.5 hours.

The catalyst is bounded at the bottom by a zone of 10 ml and at the top by a zone of 100 ml inert ceramic material. As catalyst nickle on silica gel (10% by weight Ni, Houdry type H 1170, bulk density 0.6 g per ml) is used.

The vaporous mixture is obtained by evaporation of liquid levulinic acid nitrile and mixture of the vapour with hydrogen. Per ml (bulk volume) catalyst 0.2 g levulinic acid nitrile is passed through per hour. The temperature of the catalyst is kept at 250°C by means of heating jacket round the reactor.

The composition of the vaporous mixture obtained is determined by passing the mixture through a small vessel cooled down to 0°C and analysing the condensate obtained in this process gaschromatographically. From this analysis and the weight of the levulinic acid nitrile passed over the conversion of the nitrile and the yield of 2-methylpyrrole and 5-methylpyrrolidone-2 can be calculated.

Conversion is understood to means the quantity of levulinic acid nitrile converted (quantity of nitrile passed over decreased by the quantity of nitrile in the condensate), expressed as percentages of the quantity of nitrile passed over.

The yield of 2-methylpyrrole, respectively 5-methylpyrrolidone-2, is understood to mean the quantity of 2-methylpyrrole, respectively 5-methylpyrrolidone-2, in the condensate expressed as percentages of the quantity of 2-methylpyrrole, respectively 5-methylpyrrolidone-2, which can theoretically be formed from the quantity of nitrile converted.

The conversion amounts to 98.9%. The yield of 2-methylpyrrole 61.5% and of 5-methylpyrrolidone-2 2.9%.

### Example II

Example I is repeated, using palladium and sodium on γ-aluminium oxide (0.5% by weight Pd and 0.4% by weight Na) as catalyst.

The conversion amounts to 95.5%. The yield of 2-methylpyrrole 78.8% and of 5-methylpyrrolidone-2 13.4%.

### Example III

Example I is repeated, using copper oxide on zinc oxide and γ-aluminium oxide (30% by weight CuO, 55% by weight ZnO) as catalyst. A conversion of 63.5% is reached. The yield of 2-methylpyrrole is 29% and of 5-methylpyrrolidone-2 21.6%.

### Example IV

Example I is repeated, using 2.3-dimethyl-4-oxopentanenitrile as starting compound and palladium and sodium on γ-aluminium oxide (0.5%) by weight Pd and 0.4% by weight Na) as catalyst. The conversion amounts to 91.5%. The yield of 2,3,4-trimethylpyrrole amounts to 61.3% and of 3,4,5-trimethylpyrrolidone-2 7.5%.

## Claims

1. Process for the preparation of a 2-alkylpyrrole of formula (I)

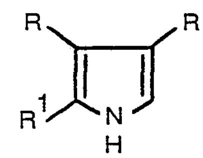

(I)

wherein $R^1$ represents an alkyl group with 1—3 C-atoms and the two Rs represent, independently of each other, H or an alkyl group with 1—2 carbon atoms by reaction of a 4-oxoalkanenitrile of formula (II)

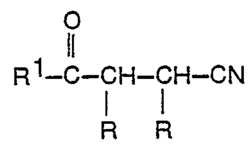

(II)

wherein $R^1$ and R have the meanings mentioned above in the presence of $H_2$ and a catalyst containing a metal selected from Cu, Ni, Pt, Pd or Rh or a compound thereof, optionally containing a promoter and optionally deposited on a supporting material at elevated temperatures, characterized in that the reaction takes place in the gas phase at a temperature of 150—350°C.

2. Process accoridng to claim 1, characterized in that the catalyst is used on aluminium oxide as support.

3. Process according to any one of claims 1—2, characterized in that an alkali metal is used as promoter.

4. Process according to any one of claims 1—3, characterized in that the contact with the catalyst is effected at a temperature of between 175 and 300°C.

5. Process according to any one of claims 1—4, characterized in that per mole starting product 1—50 moles hydrogen is used.

## Patentansprüche

1. Verfahren zur Herstellung eines 2-Alkylpyrrols der Formel (I)

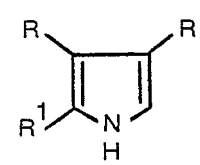

(I)

worin $R^1$ eine Alkylgruppe mit 1—3 C-Atomen

darstellt und die beiden Reste R unabhängig voneinander H oder eine Alkylgruppe mit 1—2 C-Atomen darstellen, durch Reaktion eines 4-Oxo-alkannitrils der Formel (II)

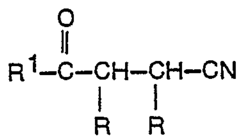

(II)

worin $R^1$ und R die oben angegebenen Bedeutungen haben, in Gegenwart von $H_2$ und einem Katalysator, der ein Metall, ausgewählt aus Cu, Ni, Pt, Pd oder Rh oder eine Verbindung davon, enthält, gegebenenfalls einen Promotor enthält und gegebenenfalls auf einem Trägermaterial abgelagert ist, bei erhöhten Temperaturen, dadurch gekennzeichnet, daß die Reaktion in der Gasphase bei einer Temperatur von 150—350°C stattfindet.

2. Verfahren nach Anspruch 1, dadurch gekennzeichnet, daß der Katalysator auf Aluminiumoxid als Träger verwendet wird.

3. Verfahren nach einem der Ansprücher 1—2, dadurch gekennzeichnet, daß ein Alkalimetall als Promotor verwendet wird.

4. Verfahren nach einem der Ansprüche 1—3, dadurch gekennzeichnet, daß der Kontakt mit dem Katalysator bei einer Temperatur zwischen 175 und 300°C bewirkt wird.

5. Verfahren nach einem der Ansprüche 1—4, dadurch gekennzeichnet, daß je Mol Ausgangsprodukt 1—50 Mol Wasserstoff verwendet werden.

## Revendications

1. Procédé pour la préparation d'une 2-alkylpyrrole de formule (I)

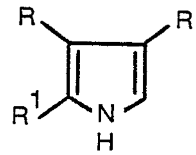

(I)

dans laquelle $R^1$ représente un radical alkyle de 1 à 3 atomes de carbone et les deux R représentent, indépendamment l'un de l'autre, H ou un radical alkyle de 1 à 2 atomes de carbone par réaction d'un 4-oxo-alcanenitrile de formule (II)

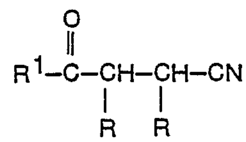

(II)

Dans laquelle $R^1$ et R ont les mêmes significations que ci-dessus, en présence de $H_2$ et d'un catalyseur contenant un métal choisi parmi Cu, Ni, Pt, Pd, ou Rh ou un composé de celui-ci, contenant facultativement un activant et facultativement déposé sur une matière de support à des températures élevées, caractérisé en ce que la réaction a lieu en phase gazeuse à une température de 150 à 350°C.

2. Procédé selon la revendication 1, caractérisé en ce qu'on utilise le catalyseur sur un support d'oxyde d'aluminium.

3. Procédé selon l'une quelconque des revendications 1 et 2, caractérisé en ce qu'on utilise comme activant un métal alcalin.

4. Procédé selon l'une quelconque des revendications 1 à 3, caractérisé en ce qu'on effectue le contact avec le catalyseur à une température de 175 à 300°C.

5. Procédé selon l'une quelconque des revendications 1 à 4, caractérisé en ce que par mole du produit de départ, on utilise 1 à 50 moles d'hydrogène.